# EUROPEAN PATENT APPLICATION

(11) **EP 3 556 760 A1**
(43) Date of publication of application: **23.10.2019**
(21) Application number: 18168269.1
(22) Date of filing: 19.04.2018
(51) Int. Cl.: C07D 471/10, C07D 513/04, A61P 35/00, A61K 31/435

(54) **SPIRO COMPOUNDS**

(71) Applicant: F. Hoffmann-La Roche AG, 4070 Basel (CH)
(72) Inventor: The designation of the inventor has not yet been filed
(74) Representative: Müller-Afraz, Simona

(57) **Abstract**

The present invention provides compounds which bind to the ubiquitously expressed E3 ligase protein cereblon (CRBN) and alter the substrate specificity of the CRBN E3 ubiquitin ligase complex, resulting in breakdown of intrinsic downstream proteins. Present compounds are thus useful for the treatment of various cancers.

## Description

### Field of the Invention

The present invention provides compounds which bind to the ubiquitously expressed E3 ligase protein cereblon (CRBN) and alter the substrate specificity of the CRBN E3 ubiquitin ligase complex, resulting in breakdown of intrinsic downstream proteins. Present compounds are thus useful for the treatment of various cancers.

### Background of the Invention

The field of targeted protein degradation promoted by small molecules has been intensively studied over the last years ¹.

Protein degradation plays a role in various cellular functions, i.e. the concentrations of regulatory proteins are adjusted through degradation into small peptides to maintain health and productivity of the cells.

Cereblon is a protein that forms an E3 ubiquitin ligase complex, which ubiquinates various other proteins. Cereblon is known as the primary target for anticancer thalidomide analogs. A higher expression of cereblon has been linked to the efficiency of thalidomide analogs in cancer therapy.

In recent years, a few compounds have been described as useful modulators of targeted ubiquitination, e.g. WO2013020557², WO2013063560³, WO 2013106643⁴, WO2015160845⁵, WO2016011906⁶, WO2016105518⁷, WO2017007612⁸, WO2017024318⁹ and WO2017117473¹⁰.

However, there is still an ongoing need for effective treatment of cancers.

### Summary of the Invention

The present invention provides spirosuccinimides (A = CH₂) and spirohydantoins (A = NH) of formula I, or a pharmaceutically acceptable salt thereof, 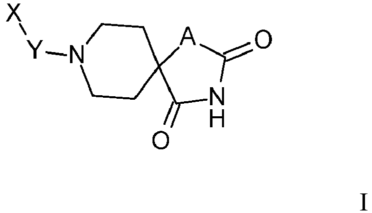 wherein the substituents and variables are as described below and in the claims, or a pharmaceutically acceptable salt thereof.

The present compounds are useful for the therapeutic and/or prophylactic treatment of cancer.

The compounds of the present invention can further be used as part of bifunctional compounds that comprise the compounds of present invention as E3 Ubiquitin Ligase moiety that is linked to a moiety that binds to a target protein where the target protein is proximate to the ubiquitin ligase to effect degradation of said protein.

### Detailed Description of the Invention

The present invention provides a compound of formula I and their pharmaceutically acceptable salts thereof, the preparation of the above mentioned compounds, medicaments containing them and their manufacture as well as the use of the above mentioned compounds in the therapeutic and/or prophylactic treatment of cancer.

The following definitions of the general terms used in the present description apply irrespectively of whether the terms in question appear alone or in combination with other groups.

Unless otherwise stated, the following terms used in this application, including the specification and claims, have the definitions given below. It must be noted that, as used in the specification and the appended claims, the singular forms "a", "an," and "the" include plural referents unless the context clearly dictates otherwise.

The term "C₁₋₆-alkyl", alone or in combination with other groups, stands for a hydrocarbon radical which may be linear or branched, with single or multiple branching, wherein the alkyl group in general comprises 1 to 6 carbon atoms, for example, methyl (Me), ethyl (Et), propyl, isopropyl (i-propyl), n-butyl, i-butyl (isobutyl), 2-butyl (sec-butyl), t-butyl (*tert*-butyl), isopentyl, 2-ethyl-propyl (2-methyl-propyl), 1,2-dimethyl-propyl and the like. A specific group is methyl.

The term "heteroaryl" denotes a monovalent heterocyclic mono- or bicyclic ring system of 5 to 12 ring atoms, comprising 1, 2, 3 or 4 heteroatoms selected from N, O and S, the remaining ring atoms being carbon and in which all rings are aromatic. Examples of heteroaryl moieties include pyrrolyl, furanyl, thienyl, imidazolyl, oxazolyl, thiazolyl, triazolyl, oxadiazolyl, thiadiazolyl, tetrazolyl, pyridinyl, pyrazinyl, pyrazolyl, pyridazinyl, pyrimidinyl, triazinyl, azepinyl, diazepinyl, isoxazolyl, benzofuranyl, isothiazolyl, benzothienyl, indolinyl, indolyl, isoindolyl, isobenzofuranyl, benzimidazolyl, benzoxazolyl, benzoisoxazolyl, benzothiazolyl, benzoisothiazolyl, benzooxadiazolyl, benzothiadiazolyl, benzotriazolyl, purinyl, quinolinyl, isoquinolinyl, dihydroquinolyl, dihydropyrrolopyridinyl, dihydronaphthyridinyl, chromanyl, tetrahydroquinolinyl, dihydrocyclopentapyridinyl quinazolinyl, or quinoxalinyl. Particular examples are pyridinyl, benzo[d]thiazolyl, thiazolo[4,5-b]pyridinyl, benzofuranyl or pyrazolo[1,5-a]pyridinyl.

The term "aryl" denotes a monovalent aromatic carbocyclic mono- or bicyclic ring system comprising 6 to 10 carbon ring atoms and in which at least one ring is aromatic. Examples of aryl moieties include phenyl (Ph), indanyl, 1,2,3,4-tetrahydronaphthalenyl and naphthyl. Particular examples are phenyl and 1,2,3,4-tetrahydronaphthalenyl.

The term "pharmaceutically acceptable" denotes an attribute of a material which is useful in preparing a pharmaceutical composition that is generally safe, non-toxic, and neither biologically nor otherwise undesirable and is acceptable for veterinary as well as human pharmaceutical use.

The term "a pharmaceutically acceptable salt" refers to a salt that is suitable for use in contact with the tissues of humans and animals. Examples of suitable salts with inorganic and organic acids are, but are not limited to, acetic acid, citric acid, formic acid, fumaric acid, hydrochloric acid, lactic acid, maleic acid, malic acid, methane-sulfonic acid, nitric acid, phosphoric acid, p-toluenesulphonic acid, succinic acid, sulfuric acid (sulphuric acid), tartaric acid, trifluoroacetic acid and the like. Particular acids are formic acid, trifluoroacetic acid and hydrochloric acid. Specific acids are hydrochloric acid, trifluoroacetic acid and fumaric acid.

The terms "pharmaceutically acceptable auxiliary substance" refer to carriers and auxiliary substances such as diluents or excipients that are compatible with the other ingredients of the formulation.

The term "pharmaceutical composition" encompasses a product comprising specified ingredients in pre-determined amounts or proportions, as well as any product that results, directly or indirectly, from combining specified ingredients in specified amounts. Particularly it encompasses a product comprising one or more active ingredients, and an optional carrier comprising inert ingredients, as well as any product that results, directly or indirectly, from combination, complexation or aggregation of any two or more of the ingredients, or from dissociation of one or more of the ingredients, or from other types of reactions or interactions of one or more of the ingredients.

"Therapeutically effective amount" means an amount of a compound that, when administered to a subject for treating a disease state, is sufficient to effect such treatment for the disease state. The "therapeutically effective amount" will vary depending on the compound, disease state being treated, the severity or the disease treated, the age and relative health of the subject, the route and form of administration, the judgment of the attending medical or veterinary practitioner, and other factors.

The term "as defined herein" and "as described herein" when referring to a variable incorporates by reference the broad definition of the variable as well as particularly, more particularly and most particularly definitions, if any.

The terms "treating", "contacting" and "reacting" when referring to a chemical reaction means adding or mixing two or more reagents under appropriate conditions to produce the indicated and/or the desired product. It should be appreciated that the reaction which produces the indicated and/or the desired product may not necessarily result directly from the combination of two reagents which were initially added, i.e., there may be one or more intermediates which are produced in the mixture which ultimately leads to the formation of the indicated and/or the desired product.

The term "aromatic" denotes the conventional idea of aromaticity as defined in the literature, in particular in IUPAC - Compendium of Chemical Terminology, 2nd, A. D. McNaught & A. Wilkinson (Eds). Blackwell Scientific Publications, Oxford (1997).

The term "pharmaceutically acceptable excipient" denotes any ingredient having no therapeutic activity and being non-toxic such as disintegrators, binders, fillers, solvents, buffers, tonicity agents, stabilizers, antioxidants, surfactants or lubricants used in formulating pharmaceutical products.

Whenever a chiral carbon is present in a chemical structure, it is intended that all stereoisomers associated with that chiral carbon are encompassed by the structure as pure stereoisomers as well as mixtures thereof.

The invention also provides pharmaceutical compositions, methods of using, and methods of preparing the aforementioned compounds.

All separate embodiments may be combined.
E1: One embodiment of the invention provides a compound of formula I, or a pharmaceutically acceptable salt thereof, 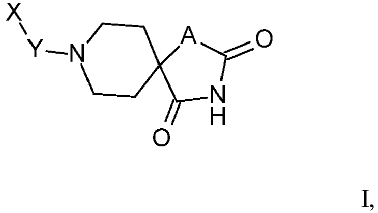 wherein
   A is selected from the group consisting of
      i.) NH, and
      ii.) CH₂,
   X is selected from the group consisting of
      i.) aryl,
      ii.) aryl substituted by R¹,
      iii.) heteroaryl, and
      iv.) heteroaryl substituted by R²;
   Y is absent or -C(=O)-;
   R¹ is selected from the group consisting of
      i.) -C(=O)-OH,
      ii.) -NH-C(=O)-C₁₋₆-alkyl,
      iii.) -NH₂, and
      iv.) -NO₂;
   R² is selected from the group consisting of
      i.) -C(=O)-OH
      ii.) -C(=O)-O-C₁₋₆-alkyl,
      iii.) -NH₂, and
      iv.) -NO₂.
E2: One embodiment of the invention provides a compound of formula I, or a pharmaceutically acceptable salt thereof, 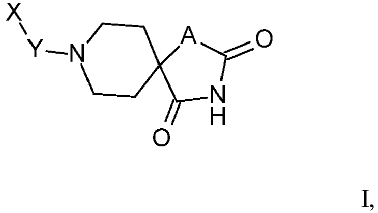 wherein
   A is selected from the group consisting of
      i.) NH, and
      ii.) CH₂,
   X is selected from the group consisting of
      i.) aryl,
      ii.) aryl substituted by R¹,
      iii.) heteroaryl, and
      iv.) heteroaryl substituted by R²;
   Y is absent or -C(=O)-;
   R¹ is selected from the group consisting of
      i.) -NH-C(=O)-C₁₋₆-alkyl, and
      ii.) -NO₂;
   R² is selected from the group consisting of
      i.) -C(=O)-O-C₁₋₆-alkyl, and
      ii.) -NO₂.
E3: A compound of formula I, or a pharmaceutically acceptable salt thereof, as described herein, wherein
   X is selected from the group consisting of
      i.) aryl, in particular 1,2,3,4-tetrahydronaphthalenyl or phenyl,
      ii.) phenyl substituted by R¹,
      iii.) heteroaryl, in particular benzo[d]thiazolyl, thiazolo[4,5-b]pyridinyl, benzofuranyl or pyrazolo[1,5-a]pyridinyl,
      iv.) pyridinyl substituted by R²;
   R¹ is selected from the group consisting of
      iii.) -NH-C(=O)-C₁₋₆-alkyl, in particular -NH-C(=O)-CH₃, and
      iv.) -NO₂;
   R² is selected from the group consisting of
      iii.) -C(=O)-O-C₁₋₆-alkyl, in particular -C(=O)-O-CH₃, and
      iv.) -NO₂.
E4: A compound of formula I, or a pharmaceutically acceptable salt thereof, as described herein, wherein A is NH.
E5: A compound of formula I, or a pharmaceutically acceptable salt thereof, as described herein, wherein A is CH₂.
E6: A compound of formula I, or a pharmaceutically acceptable salt thereof, as described herein, wherein Y is absent.
E7: A compound of formula I, or a pharmaceutically acceptable salt thereof, as described herein, wherein Y is -C(=O)-.
E8: The compound of formula I, or pharmaceutically acceptable salts thereof, as described herein, selected from the group consisting of
   (*S*)-8-(1,2,3,4-tetrahydronaphthalene-1-carbonyl)-2,8-diazaspiro[4.5]decane-1,3-dione,
   (*R*)-8-(1,2,3,4-tetrahydronaphthalene-1-carbonyl)-2,8-diazaspiro[4.5]decane-1,3-dione,
   (*S*)-8-(1,2,3,4-tetrahydronaphthalene-1-carbonyl)-1,3,8-triazaspiro[4.5]decane-2,4-dione,
   (*R*)-8-(1,2,3,4-tetrahydronaphthalene-1-carbonyl)-1,3,8-triazaspiro[4.5]decane-2,4-dione,
   8-benzoyl-1,3,8-triazaspiro[4.5]decane-2,4-dione,
   8-(benzo[*d*]thiazol-2-yl)-1,3,8-triazaspiro[4.5]decane-2,4-dione,
   8-(thiazolo[4,5-*b*]pyridin-2-yl)-1,3,8-triazaspiro[4.5]decane-2,4-dione,
   methyl 6-(2,4-dioxo-1,3,8-triazaspiro[4.5]decan-8-yl)nicotinate,
   *N*-(4-(2,4-dioxo-1,3,8-triazaspiro[4.5]decane-8-carbonyl)phenyl)acetamide,
   *N*-(3-(2,4-dioxo-1,3,8-triazaspiro[4.5]decane-8-carbonyl)phenyl)acetamide,
   8-(benzofuran-3-carbonyl)-1,3,8-triazaspiro[4.5]decane-2,4-dione,
   8-(pyrazolo[1,5-*a*]pyridine-3-carbonyl)-1,3,8-triazaspiro[4.5]decane-2,4-dione,
   8-(5-nitropyridin-2-yl)-1,3,8-triazaspiro[4.5]decane-2,4-dione,
   8-(4-nitrophenyl)-1,3,8-triazaspiro[4.5]decane-2,4-dione,
   8-(benzo[*d*]thiazole-5-carbonyl)-1,3,8-triazaspiro[4.5]decane-2,4-dione,
   8-(thiazolo[4,5-*b*]pyridin-2-yl)-2,8-diazaspiro[4.5]decane-1,3-dione,
   8-(5-nitropyridin-2-yl)-2,8-diazaspiro[4.5]decane-1,3-dione,
   8-(benzo[*d*]thiazol-2-yl)-2,8-diazaspiro[4.5]decane-1,3-dione,
   8-(4-nitrophenyl)-2,8-diazaspiro[4.5]decane-1,3-dione,
   8-benzoyl-2,8-diazaspiro[4.5]decane-1,3-dione,
   8-(benzo[*d*]thiazole-5-carbonyl)-2,8-diazaspiro[4.5]decane-1,3-dione,
   8-(benzofuran-3-carbonyl)-2,8-diazaspiro[4.5]decane-1,3-dione,
   methyl 6-(1,3-dioxo-2,8-diazaspiro[4.5]decan-8-yl)nicotinate, and
   8-(pyrazolo[1,5-*a*]pyridine-3-carbonyl)-2,8-diazaspiro[4.5]decane-1,3-dione.
E9: The compound of formula I, or pharmaceutically acceptable salts thereof, as described herein, wherein X is selected from the group consisting of
   i.) 1,2,3,4-tetrahydronaphthalenyl or phenyl,
   ii.) phenyl substituted by R¹, wherein R¹ is -NH-C(=O)-CH₃ or -NO₂,
   iii.) benzo[d]thiazolyl, thiazolo[4,5-b]pyridinyl, benzofuranyl or pyrazolo[1,5-a]pyridinyl,
   iv.) pyridinyl substituted by R², wherein R² is -C(=O)-O-CH₃ or -NO₂.
E10: The compound of formula I, or pharmaceutically acceptable salts thereof, as described herein, wherein X is aryl.
E11: The compound of formula I, or pharmaceutically acceptable salts thereof, as described herein, wherein X is phenyl.
E12: The compound of formula I, or pharmaceutically acceptable salts thereof, as described herein, wherein X is 1,2,3,4-tetrahydronaphthalenyl.
E13: The compound of formula I, or pharmaceutically acceptable salts thereof, as described herein, wherein X is aryl substituted by R¹.
E14: The compound of formula I, or pharmaceutically acceptable salts thereof, as described herein, wherein X is phenyl substituted by -NH-C(=O)-CH₃ or -NO₂.
E15: The compound of formula I, or pharmaceutically acceptable salts thereof, as described herein, wherein X is phenyl substituted by -NH-C(=O)-CH₃.
E16: The compound of formula I, or pharmaceutically acceptable salts thereof, as described herein, wherein X is phenyl substituted by -NO₂.
E17: The compound of formula I, or pharmaceutically acceptable salts thereof, as described herein, wherein X is heteroaryl.
E18: The compound of formula I, or pharmaceutically acceptable salts thereof, as described herein, wherein X is benzo[d]thiazolyl, thiazolo[4,5-b]pyridinyl, benzofuranyl or pyrazolo[1,5-a]pyridinyl.
E19: The compound of formula I, or pharmaceutically acceptable salts thereof, as described herein, wherein X is benzo[d]thiazolyl.
E20: The compound of formula I, or pharmaceutically acceptable salts thereof, as described herein, wherein X is thiazolo[4,5-b]pyridinyl.
E21: The compound of formula I, or pharmaceutically acceptable salts thereof, as described herein, wherein X is benzofuranyl.
E22: The compound of formula I, or pharmaceutically acceptable salts thereof, as described herein, wherein X is pyrazolo[1,5-a]pyridinyl.
E23: The compound of formula I, or pharmaceutically acceptable salts thereof, as described herein, wherein X is heteroaryl substituted by R².
E24: The compound of formula I, or pharmaceutically acceptable salts thereof, as described herein, wherein X is pyridinyl substituted by -C(=O)-O-CH₃ or -NO₂.
E25: The compound of formula I, or pharmaceutically acceptable salts thereof, as described herein, wherein X is pyridinyl substituted by -C(=O)-O-CH₃.
E26: The compound of formula I, or pharmaceutically acceptable salts thereof, as described herein, wherein X is pyridinyl substituted by -NO₂.
E27: A compound, or pharmaceutically acceptable salts thereof, having a chemical structure comprising:

   P-L-C

   wherein
   L is a linker group;
   C is a compound of formula I as described herein,
      wherein L is chemically linked to C; and
   P is a protein targeting moiety that binds to a target protein or a target polypeptide,
      wherein L is chemically linked to P.
E28: The compound of formula P-L-C as described herein, wherein L is selected from the group consisting of:
   i) -NHCH₂-(CH₂)₁₋₃₀-C(=O)NH-, in particular -NHCH₂-(CH₂)₆₋C(=O)NH-, and
   ii) -NH-(CH₂CH₂O)₁₋₂₅-C(=O)NH-.
E29: The compound of formula P-L-C as described herein, which is selected from the group consisting of
   i) P-NHCH₂-(CH₂)₁₋₃₀-C(=O)NH-C, in particular P-NHCH₂-(CH₂)₆₋C(=O)NH-C, and
   ii) P-NH-(CH₂CH₂O)₁₋₂₅-C(=O)NH-C.
E30: The compound of formula P-L-C as described herein, wherein P is a BRD4 inhibitor, in particular wherein P is 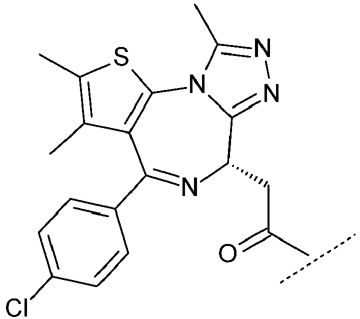
E31: A certain embodiment of the invention relates to the compound of formula I as described herein, or a pharmaceutically acceptable salt thereof, for use as therapeutically active substance.
E32: A certain embodiment of the invention relates to the compound of formula I as described herein, or a pharmaceutically acceptable salt thereof, for the use in the therapeutic and/or prophylactic treatment of cancer.
E33: A certain embodiment of the invention relates to the compound of formula I as described herein, or a pharmaceutically acceptable salt thereof, for the manufacture of a medicament for the therapeutic and/or prophylactic treatment of cancer.
E34: A certain embodiment of the invention relates to a pharmaceutical composition comprising the compound of formula I as described herein, or a pharmaceutically acceptable salt thereof, and a pharmaceutically acceptable auxiliary substance.
E35: A certain embodiment of the invention relates to a method for the therapeutic and/or prophylactic treatment of cancer, by administering the compound of formula I as described herein, or a pharmaceutically acceptable salt thereof, to a patient.
E36: A certain embodiment of the invention relates to the compound of P-L-C as described herein, or a pharmaceutically acceptable salt thereof, for use as therapeutically active substance.
E37: A certain embodiment of the invention relates to the compound of P-L-C as described herein, or a pharmaceutically acceptable salt thereof, for the use in the therapeutic and/or prophylactic treatment of cancer.
E38: A certain embodiment of the invention relates to the compound of P-L-C as described herein, or a pharmaceutically acceptable salt thereof, for the manufacture of a medicament for the therapeutic and/or prophylactic treatment of cancer.
E39: A certain embodiment of the invention relates to a pharmaceutical composition comprising the compound of P-L-C as described herein, or a pharmaceutically acceptable salt thereof, and a pharmaceutically acceptable auxiliary substance, in particular an inert carrier.
E40: A certain embodiment of the invention relates to a method for the therapeutic and/or prophylactic treatment of cancer, by administering the compound of P-L-C as described herein, or a pharmaceutically acceptable salt thereof, to a patient.

Furthermore, the invention includes all optical isomers, i.e. diastereoisomers, diastereomeric mixtures, racemic mixtures, all their corresponding enantiomers and/or tautomers as well as solvates of the compounds of formula I.

The compounds of formula I or formula P-L-C may contain one or more asymmetric centers and can therefore occur as racemates, racemic mixtures, single enantiomers, diastereomeric mixtures and individual diastereomers. Additional asymmetric centers may be present depending upon the nature of the various substituents on the molecule. Each such asymmetric center will independently produce two optical isomers and it is intended that all of the possible optical isomers and diastereomers in mixtures and as pure or partially purified compounds are included within this invention. The present invention is meant to encompass all such isomeric forms of these compounds. The independent syntheses of these diastereomers or their chromatographic separations may be achieved as known in the art by appropriate modification of the methodology disclosed herein. Their absolute stereochemistry may be determined by the x-ray crystallography of crystalline products or crystalline intermediates which are derivatized, if necessary, with a reagent containing an asymmetric center of known absolute configuration. If desired, racemic mixtures of the compounds may be separated so that the individual enantiomers are isolated. The separation can be carried out by methods well known in the art, such as the coupling of a racemic mixture of compounds to an enantiomerically pure compound to form a diastereomeric mixture, followed by separation of the individual diastereomers by standard methods, such as fractional crystallization or chromatography.

In the embodiments, where optically pure enantiomers are provided, optically pure enantiomer means that the compound contains > 90 % of the desired isomer by weight, particularly > 95 % of the desired isomer by weight, or more particularly > 99 % of the desired isomer by weight, said weight percent based upon the total weight of the isomer(s) of the compound. Chirally pure or chirally enriched compounds may be prepared by chirally selective synthesis or by separation of enantiomers. The separation of enantiomers may be carried out on the final product or alternatively on a suitable intermediate.

The compounds of formula I or formula P-L-C may be prepared in accordance with the schemes described in the examples. The starting material is commercially available or may be prepared in accordance with known methods.

The preparation of compounds of formula I of the present invention may be carried out in sequential or convergent synthetic routes. Syntheses of the compounds of the invention are shown in the following schemes 1-2 and in the description of the specific examples. The skills required for carrying out the reaction and purification of the resulting products are known to those skilled in the art. The substituents and indices used in the following description of the processes have the significance given herein before unless indicated to the contrary.

In more detail, the compounds of formula I can be manufactured by the methods given below, by the methods given in the examples or by analogous methods. Appropriate reaction conditions for the individual reaction steps are known to a person skilled in the art. The reaction sequence is not limited to the one displayed in schemes 1-2, however, depending on the starting materials and their respective reactivity the sequence of reaction steps can be freely altered. Starting materials are either commercially available or can be prepared by methods analogous to the methods given below, by methods described in references cited in the description or in the examples, or by methods known in the art.

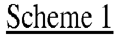

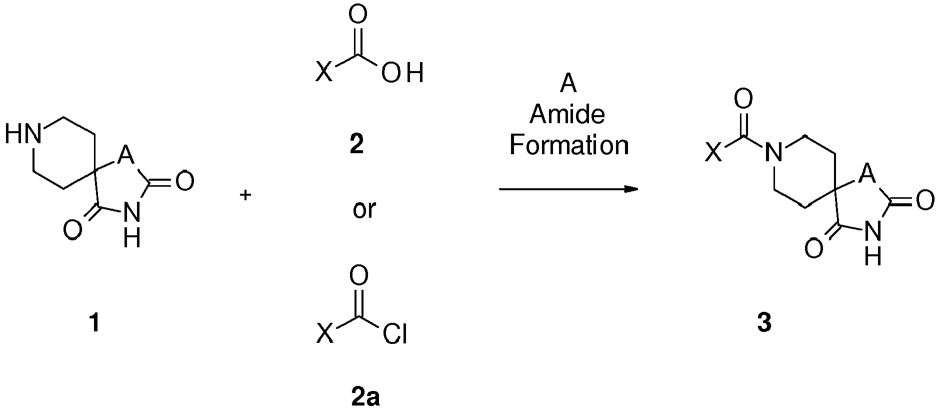

The substituents X are as described in the claims and A is NH or CH₂

***Step A*:** Amide bond formation can be accomplished by a coupling reaction between a spiro-piperidine **1** and a carboxylic acid **2** in the presence of a coupling reagent such as DCC, EDC, TBTU or HATU in the presence of an organic base such as triethylamine, *N,N-*diisopropylethylamine or N-methylmorpholine in halogenated solvents such as dichloromethane or 1,2-dichloroethane or ethereal solvents such as diethyl ether, dioxane, THF, DME or TBME or polar non-protic organic solvent such as *N*,*N*-dimethylformamide at room temperature or at elevated temperatures for 2-18 hours.

Preferred conditions are HATU with *N*,*N*-diisopropylethylamine in *N*,*N*-dimethylformamide at room temperature for 15 hours.

Examples of suitable spiro-piperidine compounds **1** include, but are not limited to, 1,3,8-triazaspiro[4.5]decane-2,4-dione ¹¹ or 2,8-diazaspiro[4.5]decane-1,3-dione ¹² , or their corresponding salts 1,3,8-triazaspiro[4.5]decane-2,4-dione hydrochloride ¹³ or 2,8-diazaspiro[4.5]decane-1,3-dione hydrochloride¹⁴.

Alternatively, amide bond formation can be accomplished by a coupling reaction between a spiro-piperidine **1** and an acyl chloride compound **2a** which has been preformed *in situ* from a carboxylic acid **2.** The acyl chloride compound **2a** can be prepared *in situ* from the corresponding carboxylic acid **2** by treatment with 1-chloro-*N*,*N*,2-trimethylpropenylamine¹⁵ in halogenated solvents such as dichloromethane or 1,2-dichloroethane, or in ethereal solvents such as diethyl ether, dioxane, THF, DME or TBME, at a temperature between 0 °C and room temperature, according to the method of Ghosez and co-workers¹⁶. Amide bond formation can then be accomplished by reaction of the acyl chloride compound **2a** with spiro-piperidine **1** in the presence of an organic base such as triethylamine, N,N-diisopropylethylamine or *N-*methylmorpholine in halogenated solvents such as dichloromethane or 1,2-dichloroethane, or in ethereal solvents such as diethyl ether, dioxane, THF, DME or TBME. Preferred conditions are N,N-diisopropylethylamine in THF at room temperature for 2 hours.

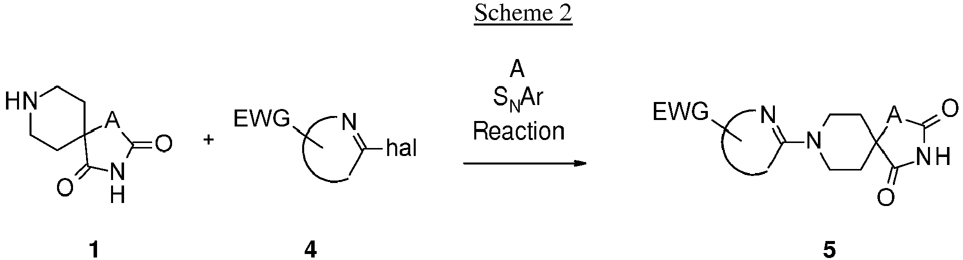

The substituents are as described in the claims, A is NH or CH₂, EWG is an electron-withdrawing group such as -CN, -CO₂R, -SO₂R or -NO₂ and hal is F or Cl.

***Step** A:* Nucleophilic aromatic substitution (S_{N}Ar) reaction can be accomplished by reaction of a spiro-piperidine **1** with an electron-deficient mono- or fused bicylic heteroaromatic compound 4 bearing a suitable leaving group such as fluorine or chlorine, in the presence of an organic base such as triethylamine, N,N-diisopropylethylamine or *N-*methylmorpholine in polar non-protic organic solvent such as *N*,*N*-dimethylformamide or N-methylpyrrolidone at elevated temperature.

Examples of suitable spiro-piperidine compounds **1** include, but are not limited to, 1,3,8-triazaspiro[4.5]decane-2,4-dione¹¹ or 2,8-diazaspiro[4.5]decane-1,3-dione¹², or their corresponding salts 1,3,8-triazaspiro[4.5]decane-2,4-dione hydrochloride¹³ or 2,8-diazaspiro[4.5]decane-1,3-dione hydrochloride¹⁴.

Examples of suitable electron-deficient heteroaromatic compounds **4** bearing a suitable leaving group include, but are not limited to, 2-chlorobenzo[d]thiazole¹⁷, 2-chlorothiazolo[4,5-b]pyridine ¹⁸, methyl 6-chloronicotinate ¹⁹, 2-chloro-5-nitropyridine ²⁰, or 1-fluoro-4-nitrobenzene²¹.

Preferred conditions are *N*,*N*-diisopropylethylamine in *N*,*N*-dimethylformamide at 120 °C for 2 hours.

### Isolation and purification of the compounds

Isolation and purification of the compounds and intermediates described herein can be effected, if desired, by any suitable separation or purification procedure such as, for example, filtration, extraction, crystallization, column chromatography, thin-layer chromatography, thick-layer chromatography, preparative low or high-pressure liquid chromatography or a combination of these procedures. Specific illustrations of suitable separation and isolation procedures can be had by reference to the preparations and examples herein below. However, other equivalent separation or isolation procedures could, of course, also be used. Racemic mixtures of chiral compounds of formula I can be separated using chiral HPLC. Racemic mixtures of chiral synthetic intermediates may also be separated using chiral HPLC.

### Salts of compounds of formula I

In cases where the compounds of formula I are basic they may be converted to a corresponding acid addition salt. The conversion is accomplished by treatment with at least a stoichiometric amount of an appropriate acid, such as hydrochloric acid, hydrobromic acid, sulfuric acid, nitric acid, phosphoric acid and the like, and organic acids such as acetic acid, propionic acid, glycolic acid, pyruvic acid, oxalic acid, malic acid, malonic acid, succinic acid, maleic acid, fumaric acid, tartaric acid, citric acid, benzoic acid, cinnamic acid, mandelic acid, methanesulfonic acid, ethanesulfonic acid, p-toluenesulfonic acid, salicylic acid and the like. Typically, the free base is dissolved in an inert organic solvent such as diethyl ether, ethyl acetate, chloroform, ethanol or methanol and the like, and the acid added in a similar solvent. The temperature is maintained between 0 °C and 50 °C. The resulting salt precipitates spontaneously or may be brought out of solution with a less polar solvent.

Insofar as their preparation is not described in the examples, the compounds of formula I or formula P-L-C as well as all intermediate products can be prepared according to analogous methods or according to the methods set forth herein. Starting materials are commercially available, known in the art or can be prepared by methods known in the art or in analogy thereto.

It will be appreciated that the compounds of general formula I or formula P-L-C in this invention may be derivatised at functional groups to provide derivatives which are capable of conversion back to the parent compound *in vivo.*

### Pharmacological Tests

The compounds of formula I or formula P-L-C and their pharmaceutically acceptable salts possess valuable pharmacological properties. The compounds were investigated in accordance with the test given hereinafter.

### Dual Fluorescent Reporter Assay

In order to measure BRD4 protein abundance in a mammalian cell system at medium throughput, a dual fluorescent reporter system was developed based on a principle described in ¹. Transient expression vectors were designed that contain the BRD4 coding sequence (NM_058243.2) fused to a fluorescent tag. Vectors were synthesized at ATUM (Newark, CA, USA) using the pD2610 CMV backbone and were built up as follows: c-terminal version BRD4_eGFP - IRES - FresnoRFP_NLS, n-terminal version eGFP_BRD4 - IRES - FresnoRFP_NLS, empty vector control eGFP - IRES - FresnoRFP_NLS. The c-terminal version was used for the reporter assays, as it presented with the best assay window. HEK293A cells (Invitrogen, Cat. No. R705-07) were cultured in Dulbecco's Modified Eagle Medium (DMEM), 10% fetal calf serum, 2 mM L-Glutamine, 1% Penicillin/Streptomycin. Transfections of the plasmids were performed with Lipofectamine 2000 according to the manufacturer's protocol (Invitrogen, Carlsbad, CA, USA). 40 hours after transfection, cells were seeded at a density of 40'000 / 100 µl / 96well flat-bottom and 8 hours later treated with compounds (stocks 10 mM in DMSO) at a 10-point dilution ranging from 0 - 25 µM. After 16 hours of treatment, cells were washed with PBS, resuspended in Accumax solution (Sigma-Aldrich Cat. No. A7089) and analyzed by flow-cytometry (CytoFlex S, BeckmanCoulter). Single cells were gated based on their forward and side-scatter profiles and pulse-width was used to exclude doublets. A minimum of 20'000 cells was acquired per sample. Analysis was performed with the program Flow Jo V10.1 on BRD4-eGFP low/medium cells (< 10⁶ FL1-A Mean Fluorescence Intensity (MFI)). A factor was derived to normalize BRD4-eGFP values to the RFP protein abundance control (20 x FL1A-GFP / FL11A-RFP), then Median and Mode values were calculated and used for comparisons between treatment conditions.

### Capillary-based Immunoassays to measure endogenous BRD4 levels

The biological activity of selected compounds (cut-off > 20% reduction in BRD4-eGFP levels) was confirmed in an additional assay which allowed the quantification of endogenous BRD4 levels. To this end, HEK293A cells (origin and culture conditions see above) were seeded at 400'000 / 300 µl / 48 well and were treated 6 hours later with compound concentrations as indicated for. 16 hours after the treatment, the cells were washed with PBS and lysed in 50 µl of UREA lysis buffer (10 mM Tris-HCl pH 8, 2% CHAPS, 7M UREA, 0.4% DTT), supplemented with 1 × protease inhibitor cocktail (Complete Mini, Roche) and 1 x phosphatase inhibitor cocktail (PhosSTOP, Sigma-Aldrich). Samples were then analyzed by Peggy Sue or WES capillary-based immunoassay systems according to the manufacturer's protocol (Protein Simple / Bio-Techne, San Jose, California, 95134 USA). Antibodies used were anti- BRD4 (Cell signaling, CST 13440 1:50) and anti-Vinculin (Sigma, V9131, 1:4000). To quantify BRD4 protein levels, the peak signal areas were normalized to the vinculin loading control and to the DMSO condition.

Further, please see Yen *et al.*²²*.*

### Fluorescence Direct Binding Protocol

### Principle

Determination of the affinities of compounds to protein containing one or more tryptophan is measurable by monitoring the fluorescence emission in direct mode. The measurements depending on the protein available amounts are performed either manually in a cuvette on ISS-PC1 photon counting spectrofluorometer or automatically in well plates on a fluorescence plate reader device. Fluorescence titrations are performed at 20 °C in the chosen binding assay buffer by using a defined constant protein concentration against ligand concentration variations. Small aliquots of known ligand concentration solubilized in DMSO were added and the fluorescence, excited at 280 nm, was recorded at 340 nm. The fluorescence intensity was corrected for protein dilution and for the filter effect (Birdsall *et al.* ²³). The corrected fluorescence intensity was plotted against the ligand concentration and fitted using a four-parameter sigmoidal function, from which the equilibrium dissociation constant K_{d} was computed using the law of mass action assuming a 1:1 protein-ligand complex (Eftink, 1997²⁴).

### Process

1) Optimization of measurement parameters to minimize protein consumption and to minimize the dilution effect and the DMSO content
2) Titration measurements of the protein against ligand by at least 12 titration steps to obtain a good s-curve fit
3) Repeat the same titration measurements with the ligand alone to enable correction
4) Check the stability of the protein once by titration against DMSO alone
5) Determination of the molar extinction coefficients of the ligand at 280 and 340 nm with help of an UV- spectrophotometer
6) Use Excel template for the correction of the measured raw data
7) Use GraphPad Prism software for the quadratic binding fit and the KD evaluation.

### Experimental Details

**Table 1: Protein - buffers, Reference compound: thalidomide**

| **Protein Batch #** | **Cereblon_17_13** |
|---|---|
| Construct name | hCereblon(M1-L442)_hDDB1(M1-H1140) |
| Concentration | 2.54 mg/ml |
| MW | 180180 Da |
| Molar extinction coefficient | ε₂₈₀ = 165045 M⁻¹.cm⁻¹ |
| Storage buffer | 20 mM MES pH 6.5 200 mM NaCl 1 mM TCEP |
| Assay buffer | 50 mM Hepes 7.4 200 mM NaCl |

**Table 2: Settings**

| | |
|---|---|
| **Device** | ISS -PC 1 |
| Excitation wavelength [nm] | 280 |
| Emission wavelength [nm] | 340 |
| Cuvette | Hellma 115F-QS |
| Volume [µL] | 500 |

### Protein preparation :

**Table 3: Protein preparation**

| Volume Protein [µL] | Volume buffer [µL] | Protein concentration [M] |
|---|---|---|
| 1.8 @ 2.54 mg/ml | 498.2 | 5.0 E-8 |

**Table 4: Titration steps**

| **C Lig [M]** | **C Aliquot [M]** | **V Aliquot [µL]** | **C Prot[M]** | **Dilution factor** |
|---|---|---|---|---|
| 1E-10 | 1.0E-07 | 0.5 | 4.995E-08 | 1.001 |
| 1.1E-09 | 1.0E-06 | 0.5 | 4.990E-08 | 1.002 |
| 3.1E-09 | 1.0E-06 | 1 | 4.980E-08 | 1.004 |
| 5.1E-09 | 1.0E-06 | 1 | 4.970E-08 | 1.006 |
| 1.51E-08 | 1.0E-05 | 0.5 | 4.965E-08 | 1.007 |
| 2.51E-08 | 1.0E-05 | 0.5 | 4.960E-08 | 1.008 |
| 4.51E-08 | 1.0E-05 | 1 | 4.950E-08 | 1.01 |
| 6.51E-08 | 1.0E-05 | 1 | 4.941E-08 | 1.012 |
| 1.651E-07 | 1.0E-04 | 0.5 | 4.936E-08 | 1.013 |
| 3.651E-07 | 1.0E-04 | 1 | 4.926E-08 | 1.015 |
| 5.651E-07 | 1.0E-04 | 1 | 4.916E-08 | 1.017 |
| 7.651E-07 | 1.0E-04 | 1 | 4.907E-08 | 1.019 |
| 9.651E-07 | 1.0E-04 | 1 | 4.897E-08 | 1.021 |
| 1.9651E-06 | 1.0E-03 | 0.5 | 4.892E-08 | 1.022 |
| 2.9651E-06 | 1.0E-03 | 0.5 | 4.888E-08 | 1.023 |
| 1.29651E-05 | 1.0E-02 | 0.5 | 4.883E-08 | 1.024 |
| 2.29651E-05 | 1.0E-02 | 0.5 | 4.878E-08 | 1.025 |
| 4.29651E-05 | 1.0E-02 | 1 | 4.869E-08 | 1.027 |
| 6.29651E-05 | 1.0E-02 | 1 | 4.859E-08 | 1.029 |
| 8.29651E-05 | 1.0E-02 | 1 | 4.850E-08 | 1.031 |

**Table 5: affinities of examples to protein**

| **Ex.** | **Name** | **Structure** | **Fluorescence h-Cereblon_DDB1 Mean K_{d}_EQ (µM)** |
|---|---|---|---|
| 1 | (*S*)-8-(1,2,3,4-tetrahydronaphthalen e-1-carbonyl)-1,3,8-triazaspiro[4.5]decan e-2,4-dione | 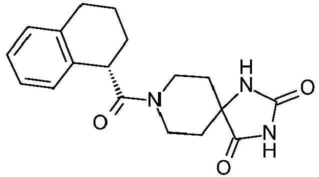 | 0.004 |
| 2 | (*R*)-8-(1,2,3,4-tetrahydronaphthalen e-1-carbonyl)-1,3,8-triazaspiro[4.5]decan e-2,4-dione | 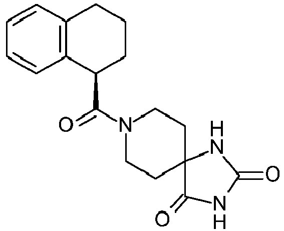 | 0.005 |
| 3 | 8-benzoyl-1,3,8-triazaspiro[4.5]decan e-2,4-dione | 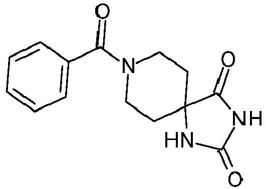 | 0.002 |
| 4 | 8-(benzo[d]thiazol-2-yl)-1,3,8-triazaspiro[4.5]decan e-2,4-dione | 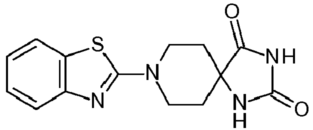 | 0.002 |
| 5 | 8-(thiazolo[4,5-b]pyridin-2-yl)-1,3,8-triazaspiro[4.5]decan e-2,4-dione | 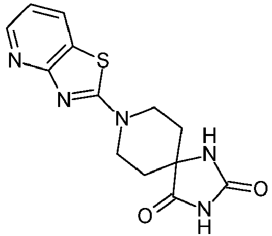 | 0.017 |
| 6 | methyl 6-(2,4-dioxo-1,3,8-triazaspiro[4.5] decan-8-yl)nicotinate | 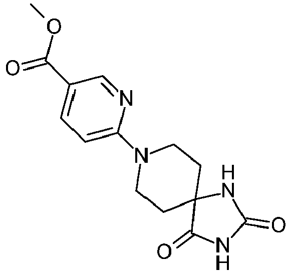 | 0.034 |
| 7 | *N*-(4-(2,4-dioxo-1,3,8-triazaspiro[4.5]decan e-8-carbonyl)phenyl)acet amide | 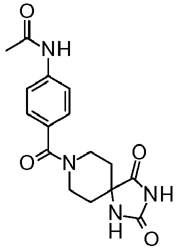 | 0.027 |
| 8 | N-(3-(2,4-dioxo-1,3,8-triazaspiro[4.5]decan e-8-carbonyl)phenyl)acet amide | 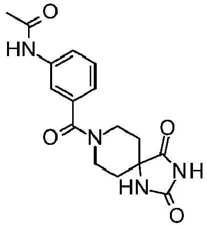 | 0.021 |
| 9 | 8-(benzofuran - 3-carbonyl)-1,3,8-triazaspiro[4.5]decan e-2,4-dione | 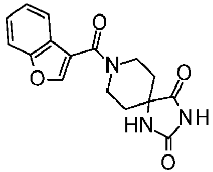 | 0.001 |
| 10 | 8-(pyrazolo[1,5-a]pyridine-3-carbonyl)-1,3,8-triazaspiro[4.5]decan e-2,4-dione | 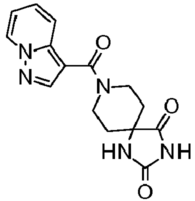 | <0.001 |
| 11 | 8-(thiazolo[4,5-b]pyridin-2-yl)-2,8-diazaspiro[4.5]decane -1,3-dione | 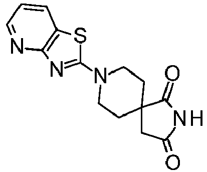 | 0.016 |
| 12 | 8-(5-nitropyridin-2-yl)-1,3,8-triazaspiro[4.5]decan e-2,4-dione | 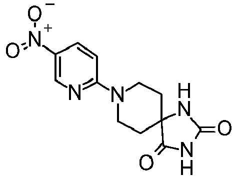 | 0.007 |
| 13 | 8-(5-nitropyridin-2-yl)-2,8-diazaspiro[4.5]decane -1,3-dione | 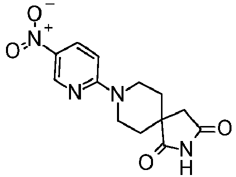 | 0.023 |
| 14 | 8-(benzo[d]thiazol-2-yl)-2,8-diazaspiro[4.5]decane -1,3-dione | 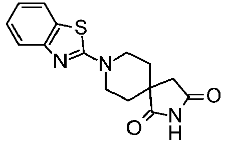 | 0.002 |
| 15 | 8-(4-nitrophenyl) - 1,3,8-triazaspiro[4.5]decan e-2,4-dione | 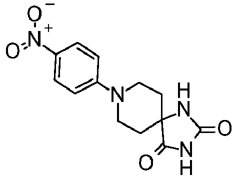 | <0.001 |
| 16 | 8-(4-nitrophenyl)-2,8-diazaspiro[4.5]decane -1,3-dione | 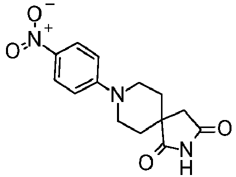 | 0.006 |
| 17 | methyl 6-(1,3-dioxo-2,8-diazaspiro[4.5]decan-8-yl)nicotinate | 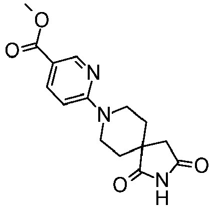 | 0.011 |
| 18 | 8-benzoyl-2,8-diazaspiro[4.5]decane -1,3-dione | 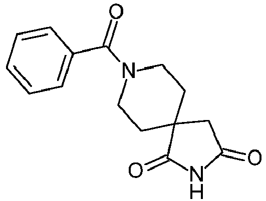 | <0.001 |
| 19 | 8-(benzo[d] thiazole-5-carbonyl)-2,8-diazaspiro[4.5]decane -1,3-dione | 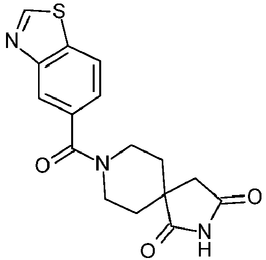 | 0.015 |
| 20 | (*S*)-8-(1,2,3,4-tetrahydronaphthalen e-1-carbonyl)-2,8-diazaspiro[4.5]decane -1,3-dione [OR ENANTIOMER] | 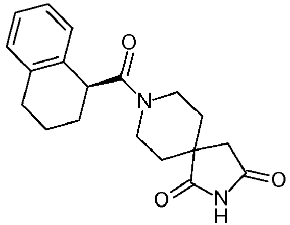 | 0.006 |
| 21 | (*R*)-8-(1,2,3,4-tetrahydronaphthalen e-1-carbonyl)-2,8-diazaspiro[4.5]decane -1,3-dione [OR ENANTIOMER] | 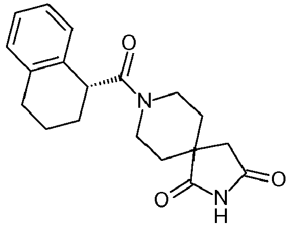 | 0.004 |
| 22 | 8-(pyrazolo[1,5-a]pyridine-3-carbonyl)-2,8-diazaspiro[4.5]decane -1,3-dione | 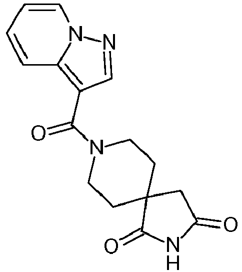 | 0.028 |
| 23 | 8-(benzofuran-3-carbonyl)-2,8-diazaspiro[4.5]decane -1,3-dione | 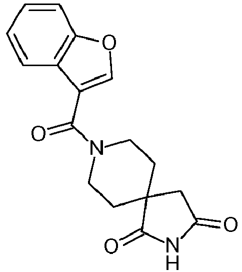 | 0.006 |
| 24 | 8-(benzo [d] thiazole-5-carbonyl)-1,3,8-triazaspiro[4.5]decan e-2,4-dione | 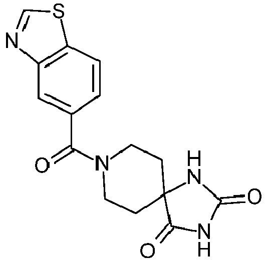 | 0.0060 |

### Pharmaceutical Compositions

The compounds of formula I or formula P-L-C and the pharmaceutically acceptable salts can be used as therapeutically active substances, e.g. in the form of pharmaceutical preparations. The pharmaceutical preparations can be administered orally, e.g. in the form of tablets, coated tablets, dragées, hard and soft gelatin capsules, solutions, emulsions or suspensions. The administration can, however, also be effected rectally, e.g. in the form of suppositories, or parenterally, e.g. in the form of injection solutions.

The compounds of formula I or formula P-L-C and the pharmaceutically acceptable salts thereof can be processed with pharmaceutically inert, inorganic or organic carriers for the production of pharmaceutical preparations. Lactose, corn starch or derivatives thereof, talc, stearic acids or its salts and the like can be used, for example, as such carriers for tablets, coated tablets, dragées and hard gelatin capsules. Suitable carriers for soft gelatin capsules are, for example, vegetable oils, waxes, fats, semi-solid and liquid polyols and the like. Depending on the nature of the active substance no carriers are however usually required in the case of soft gelatin capsules. Suitable carriers for the production of solutions and syrups are, for example, water, polyols, glycerol, vegetable oil and the like. Suitable carriers for suppositories are, for example, natural or hardened oils, waxes, fats, semi-liquid or liquid polyols and the like.

The pharmaceutical preparations can, moreover, contain pharmaceutically acceptable auxiliary substances such as preservatives, solubilizers, stabilizers, wetting agents, emulsifiers, sweeteners, colorants, flavorants, salts for varying the osmotic pressure, buffers, masking agents or antioxidants. They can also contain still other therapeutically valuable substances.

Medicaments containing a compound of formula I or formula P-L-C or a pharmaceutically acceptable salt thereof and a therapeutically inert carrier are also provided by the present invention, as is a process for their production, which comprises bringing one or more compounds of formula I and/or pharmaceutically acceptable salts thereof and, if desired, one or more other therapeutically valuable substances into a galenical administration form together with one or more therapeutically inert carriers.

The dosage can vary within wide limits and will, of course, have to be adjusted to the individual requirements in each particular case. In the case of oral administration, the dosage for adults can vary from about 0.01 mg to about 1000 mg per day of a compound of general formula I or of the corresponding amount of a pharmaceutically acceptable salt thereof. The daily dosage may be administered as single dose or in divided doses and, in addition, the upper limit can also be exceeded when this is found to be indicated.

The following examples illustrate the present invention without limiting it, but serve merely as representative thereof. The pharmaceutical preparations conveniently contain about 1-500 mg, particularly 1-100 mg, of a compound of formula I or formula P-L-C. Examples of compositions according to the invention are:

### Example A

Tablets of the following composition are manufactured in the usual manner:

**Table 6: possible tablet composition**

| ingredient | mg/tablet | | | |
|---|---|---|---|---|
| | 5 | 25 | 100 | 500 |
| Compound of formula I or formula P-L-C | 5 | 25 | 100 | 500 |
| Lactose Anhydrous DTG | 125 | 105 | 30 | 150 |
| Sta-Rx 1500 | 6 | 6 | 6 | 60 |
| Microcrystalline Cellulose | 30 | 30 | 30 | 450 |
| Magnesium Stearate | 1 | 1 | 1 | 1 |
| Total | 167 | 167 | 167 | 831 |

### Manufacturing Procedure

1. Mix ingredients 1, 2, 3 and 4 and granulate with purified water.
2. Dry the granules at 50 °C.
3. Pass the granules through suitable milling equipment.
4. Add ingredient 5 and mix for three minutes; compress on a suitable press.

### Example B-1

Capsules of the following composition are manufactured:

**Table 7: possible capsule ingredient composition**

| ingredient | mg/capsule | | | |
|---|---|---|---|---|
| | 5 | 25 | 100 | 500 |
| Compound of formula I or formula P-L-C | 5 | 25 | 100 | 500 |
| Hydrous Lactose | 159 | 123 | 148 | - |
| Corn Starch | 25 | 35 | 40 | 70 |
| Talk | 10 | 15 | 10 | 25 |
| Magnesium Stearate | 1 | 2 | 2 | 5 |
| Total | 200 | 200 | 300 | 600 |

### Manufacturing Procedure

1. Mix ingredients 1, 2 and 3 in a suitable mixer for 30 minutes.
2. Add ingredients 4 and 5 and mix for 3 minutes.
3. Fill into a suitable capsule.

The compound of formula I or formula P-L-C, lactose and corn starch are firstly mixed in a mixer and then in a comminuting machine. The mixture is returned to the mixer; the talc is added thereto and mixed thoroughly. The mixture is filled by machine into suitable capsules, e.g. hard gelatin capsules.

### Example B-2

Soft Gelatin Capsules of the following composition are manufactured:

**Table 8: possible soft gelatin capsule ingredient composition**

| ingredient | mg/capsule |
|---|---|
| Compound of formula I or formula P-L-C | 5 |
| Yellow wax | 8 |
| Hydrogenated Soya bean oil | 8 |
| Partially hydrogenated plant oils | 34 |
| Soya bean oil | 110 |
| Total | 165 |

**Table 9: possible soft gelatin capsule composition**

| ingredient | mg/capsule |
|---|---|
| Gelatin | 75 |
| Glycerol 85 % | 32 |
| Karion 83 | 8 (dry matter) |
| Titan dioxide | 0.4 |
| Iron oxide yellow | 1.1 |
| Total | 116.5 |

### Manufacturing Procedure

The compound of formula I or formula P-L-C is dissolved in a warm melting of the other ingredients and the mixture is filled into soft gelatin capsules of appropriate size. The filled soft gelatin capsules are treated according to the usual procedures.

### Example C

Suppositories of the following composition are manufactured:

**Table 10: possible suppository composition**

| ingredient | mg/supp. |
|---|---|
| Compound of formula I or formula P-L-C | 15 |
| Suppository mass | 1285 |
| Total | 1300 |

### Manufacturing Procedure

The suppository mass is melted in a glass or steel vessel, mixed thoroughly and cooled to 45 °C. Thereupon, the finely powdered compound of formula I or formula P-L-C is added thereto and stirred until it has dispersed completely. The mixture is poured into suppository moulds of suitable size, left to cool; the suppositories are then removed from the moulds and packed individually in wax paper or metal foil.

### Example D

Injection solutions of the following composition are manufactured:

**Table 11: possible injection solution composition**

| ingredient | mg/injection solution. |
|---|---|
| Compound of formula I or formula P-L-C | 3 |
| Polyethylene Glycol 400 | 150 |
| acetic acid | q.s. ad pH 5.0 |
| water for injection solutions | ad 1.0 ml |

### Manufacturing Procedure

The compound of formula I or formula P-L-C is dissolved in a mixture of Polyethylene Glycol 400 and water for injection (part). The pH is adjusted to 5.0 by addition of acetic acid. The volume is adjusted to 1.0 ml by addition of the residual amount of water. The solution is filtered, filled into vials using an appropriate overage and sterilized.

### Example E

Sachets of the following composition are manufactured:

**Table 12: possible sachet composition**

| ingredient | mg/sachet |
|---|---|
| Compound of formula I or formula P-L-C | 50 |
| Lactose, fine powder | 1015 |
| Microcrystalline cellulose (AVICEL PH 102) | 1400 |
| Sodium carboxymethyl cellulose | 14 |
| Polyvinylpyrrolidon K 30 | 10 |
| Magnesium stearate | 10 |
| Flavoring additives | 1 |
| Total | 2500 |

### Manufacturing Procedure

The compound of formula I or formula P-L-C is mixed with lactose, microcrystalline cellulose and sodium carboxymethyl cellulose and granulated with a mixture of polyvinylpyrrolidone in water. The granulate is mixed with magnesium stearate and the flavoring additives and filled into sachets.

### Experimental Part

The following examples are provided for illustration of the invention. They should not be considered as limiting the scope of the invention, but merely as being representative thereof.

### Example 1

### (S)-8-(1,2,3,4-Tetrahydronaphthalene-1-carbonyl)-1,3,8-triazaspiro[4.5]decane-2,4-dione

To a solution of 1,3,8-triazaspiro[4.5]decane-2,4-dione¹¹ (100 mg, 591 µmol, Eq: 1) and (*S*)-1,2,3,4-tetrahydronaphthalene-1-carboxylic acid²⁵ (109 mg, 621 µmol, Eq: 1.05) in *N,N-*dimethylformamide (1ml) were added HATU (449 mg, 1.18 mmol, Eq: 2.0) and DIPEA (229 mg, 310 µl, 1.77 mmol, Eq: 3.0) at room temperature. The reaction mixture was stirred for 15 hours and was then partitioned between ethyl acetate (50 ml) and water (30 ml). The layers were separated. The aqueous layer was extracted with ethyl acetate (2 x 20 ml). The combined organic layers were washed with brine (30 ml), dried over anhydrous sodium sulfate and concentrated *in vacuo.* The crude material was purified by reversed phase HPLC followed by lyophilisation to afford
(*S*)-8-(1,2,3,4-tetrahydronaphthalene-1-carbonyl)-1,3,8-triazaspiro[4.5]decane-2,4-dione (45 mg, 137 µmol, 23.3 %) as a white solid. MS (ISP): 328.0 ([M+H]⁺).

### Example 2

### (R)-8-(1,2,3,4-Tetrahydronaphthalene-1-carbonyl)-1,3,8-triazaspiro[4.5]decane-2,4-dione

The title compound was obtained in analogy to example 1 using (R)-1,2,3,4-tetrahydronaphthalene-1-carboxylic acid ²⁶ in place of (*S*)-1,2,3,4-tetrahydronaphthalene-1-carboxylic acid. White solid. MS (ISP): 328.0 ([M+H]⁺).

### Example 3

### 8-Benzoyl-1,3,8-triazaspiro[4.5]decane-2,4-dione

The title compound was obtained in analogy to example 1 using benzoic acid²⁷ in place of (*S*)-1,2,3,4-tetrahydronaphthalene-1-carboxylic acid. White solid. MS (ISP): 274.1 ([M+H]⁺).

### Example 4

### 8-(Benzo[d]thiazol-2-yl)-1,3,8-triazaspiro[4.5]decane-2,4-dione

To a suspension of 1,3,8-triazaspiro[4.5]decane-2,4-dione¹¹ (20 mg, 118 µmol, Eq: 1) and DIPEA (45.8 mg, 61.9 µl, 355 µmol, Eq: 3) in *N*,*N*-dimethylformamide (0.5 ml) was added 2-chlorobenzo[d]thiazole (22.1 mg, 130 µmol, Eq: 1.1). The reaction mixture was stirred at 120 °C for 2 h. The reaction mixture was poured into a mixture of ethylacetate/tetrahydrofuran (1:1), then the organic layer was washed with water followed with brine, dried over Na₂SO₄ and concentrated *in vacuo.* The crude material was purified by flash column chromatography (silica gel, eluent: 0 to 5% of methanol in dichloromethane) to afford 8-(benzo[d]thiazol-2-yl)-1,3,8-triazaspiro[4.5]decane-2,4-dione (11 mg, 36.4 µmol, 30.8 %) as a white solid. MS (ISP): 303.1 ([M+H]⁺).

### Example 5

### 8-(Thiazolo[4,5-b]pyridin-2-yl)-1,3,8-triazaspiro[4.5]decane-2,4-dione

The title compound was obtained in analogy to example 4 using 2-chlorothiazolo[4,5-b]pyridine¹⁸ in place of 2-chlorobenzo[d]thiazole. Brown solid. MS (ISP): 304.1 ([M+H]⁺).

### Example 6

### Methyl 6-(2,4-dioxo-1,3,8-triazaspiro[4.5]decan-8-yl)nicotinate

The title compound was obtained in analogy to example 4 using methyl 6-chloronicotinate¹⁹ in place of 2-chlorobenzo[d]thiazole and stirring for 16 hours instead of 2 hours. Brown solid. MS (ISP): 305.1 ([M+H]⁺).

### Example 7

### N-(4-(2,4-Dioxo-1,3,8-triazaspiro[4.5]decane-8-carbonyl)phenyl)acetamide

To a solution of 1,3,8-triazaspiro[4.5]decane-2,4-dione (36 mg, 213 µmol, Eq: 1) and 4-acetamidobenzoic acid²⁸ (40 mg, 223 µmol, Eq: 1.05) in *N*,*N*-dimethylformamide (1 ml) were added DIPEA (110 mg, 149 µl, 851 µmol, Eq: 4) and HATU (121 mg, 319 µmol, Eq: 1.5). The reaction mixture was stirred at room temperature overnight. The reaction mixture was poured into ethyl acetate/tetrahydrofuran (1:1) and extracted with water followed with brine. The organic layer was dried over Na₂SO₄ and concentrated *in vacuo.* The crude material was purified by flash chromatography (silica gel, 4 g, 0% to 10% methanol in dichloromethane) to afford *N-*(4-(2,4-dioxo-1,3,8-triazaspiro[4.5]decane-8-carbonyl)phenyl)acetamide (10 mg, 30.3 µmol, 14.2 %) as a white solid. MS (ISP): 331.1 ([M+H]⁺).

### Example 8

### N-(3-(2,4-Dioxo-1,3,8-triazaspiro[4.5]decane-8-carbonyl)phenyl)acetamide

The title compound was obtained in analogy to example 7 using 3-acetamidobenzoic acid²⁹ in place of 4-acetamidobenzoic acid. White solid. MS (ISP): 329.1 ([M-H]⁻).

### Example 9

### 8-(Benzofuran-3-carbonyl)-1,3,8-triazaspiro[4.5]decane-2,4-dione

To a solution of 1,3,8-triazaspiro[4.5]decane-2,4-dione hydrochloride¹³ (30 mg, 146 µmol, Eq: 1) and benzofuran-3-carboxylic acid³⁰ (31.6 mg, 195 µmol, Eq: 1.1) in *N,N-*dimethylformamide (0.5 ml) were added DIPEA (68.8 mg, 92.9 µl, 532 µmol, Eq: 3) and HATU (169 mg, 443 µmol, Eq: 2.5). The reaction mixture was stirred at room temperature for 2 hours and was then poured into ethyl acetate/tetrahydrofuran (2:1) (30 ml). The aqueous layer was extracted with two portions of ethyl acetate (2 x 20 ml). The combined organic layer was dried over Na₂SO₄ and concentrated *in vacuo.* The crude material was purified by flash chromatography (silica gel, 4 g, 0% to 10% methanol in dichloromethane) followed by a preparative RP-HPLC and lyophilisation to afford 8-(benzofuran-3-carbonyl)-1,3,8-triazaspiro[4.5]decane-2,4-dione (3.3 mg, 10.5 µmol, 7.22 %) as a yellow solid. MS (ISP): 314.1 ([M+H]⁺).

### Example 10

### 8-(Pyrazolo[1,5-a]pyridine-3-carbonyl)-1,3,8-triazaspiro[4.5]decane-2,4-dione

The title compound was obtained in analogy to example 9 using pyrazolo[1,5-a]pyridine-3-carboxylic acid³¹ in place of benzofuran-3-carboxylic acid. White solid. MS (ISP): 314 ([M+H]⁺).

### Example 11

### 8-(Thiazolo[4,5-b]pyridin-2-yl)-2,8-diazaspiro[4.5]decane-1,3-dione

The title compound was obtained in analogy to example 4 using 2-chlorothiazolo[4,5-b]pyridine¹⁸ in place of 2-chlorobenzo[d]thiazole and using 2,8-diazaspiro[4.5]decane-1,3-dione hydrochloride¹⁴ instead of 1,3,8-triazaspiro[4.5]decane-2,4-dione. Grey solid. MS (ISP): 303.1 ([M+H]⁺).

### Example 12

### 8-(5-Nitropyridin-2-yl)-1,3,8-triazaspiro[4.5]decane-2,4-dione

The title compound was obtained in analogy to example 4 using 2-chloro-5-nitropyridine²⁰ in place of 2-chlorobenzo[d]thiazole. Yellow solid. MS (ISP): 292.1 ([M+H]⁺).

### Example 13

### 8-(5-Nitropyridin-2-yl)-2,8-diazaspiro[4.5]decane-1,3-dione

The title compound was obtained in analogy to example 4 using 2-chloro-5-nitropyridine²⁰ in place of 2-chlorobenzo[d]thiazole and using 2,8-diazaspiro[4.5]decane-1,3-dione hydrochloride¹⁴ instead of 1,3,8-triazaspiro[4.5]decane-2,4-dione. Grey solid. MS (ISP): 291.1 ([M+H]⁺).

### Example 14

### 8-(Benzo[d]thiazol-2-yl)-2,8-diazaspiro[4.5]decane-1,3-dione

The title compound was obtained in analogy to example 4 using 2,8-diazaspiro[4.5]decane-1,3-dione hydrochloride¹⁴ instead of 1,3,8-triazaspiro[4.5]decane-2,4-dione. Grey solid. MS (ISP): 302.0 ([M+H]⁺).

### Example 15

### 8-(4-Nitrophenyl)-1,3,8-triazaspiro[4.5]decane-2,4-dione

The title compound was obtained in analogy to example 4 using 1-fluoro-4-nitrobenzene²¹ in place of 2-chlorobenzo[d]thiazole. Yellow solid. MS (ISP): 291.0 ([M+H]⁺).

### Example 16

### 8-(4-Nitrophenyl)-2,8-diazaspiro[4.5]decane-1,3-dione

The title compound was obtained in analogy to example 4 using 1-fluoro-4-nitrobenzene²¹ in place of 2-chlorobenzo[d]thiazole and using 2,8-diazaspiro[4.5]decane-1,3-dione hydrochloride¹⁴ instead of 1,3,8-triazaspiro[4.5]decane-2,4-dione. Grey solid. MS (ISP): 291.1 ([M+H]⁺).

### Example 17

### Methyl 6-(1,3-dioxo-2,8-diazaspiro[4.5]decan-8-yl)nicotinate

The title compound was obtained in analogy to example 4 using methyl 6-chloronicotinate¹⁹ in place of 2-chlorobenzo[d]thiazole and using 2,8-diazaspiro[4.5]decane-1,3-dione hydrochloride¹⁴ instead of 1,3,8-triazaspiro[4.5]decane-2,4-dione. Off-white solid. MS (ISP): 304.1 ([M+H]⁺).

### Example 18

### 8-Benzoyl-2,8-diazaspiro[4.5]decane-1,3-dione

To a solution of 2,8-diazaspiro[4.5]decane-1,3-dione hydrochloride (60 mg, 293 µmol, Eq: 1) and triethylamine (89 mg, 123 µl, 880 µmol, Eq: 3) in tetrahydrofuran (1.5 ml) was added benzoyl chloride³² (49.5 mg, 40.9 µl, 352 µmol, Eq: 1.2) at 5 °C. The reaction mixture was stirred for 3 hours. LC/MS showed the reaction was finished. The reaction mixture was partitioned between ethyl acetate/tetrahydrofuran 1:1 (20 ml) and brine (25 ml). The layers were separated. The aqueous layer was extracted with one portion of ethyl acetate (15 ml). The combined organic layers were dried over anhydrous sodium sulfate and concentrated *in vacuo.* The crude material was purified by flash chromatography (silica gel, 4 g, 0% to 10% methanol in dichloromethane) to afford 8-benzoyl-2,8-diazaspiro[4.5]decane-1,3-dione (25.2 mg, 92.5 µmol, 31.6 %) as a white solid. MS (ISP): 273.1 ([M+H]⁺).

### Example 19

### 8-(benzo[d]thiazole-5-carbonyl)-2,8-diazaspiro[4.5]decane-1,3-dione

To a suspension of benzo[d]thiazole-5-carboxylic acid (55.2 mg, 308 µmol, Eq: 1.05) in tetrahydrofuran (1 ml) was added 1-chloro-*N*,*N*,2-trimethyl-1-propenylamine (47 mg, 46.5 µl, 352 µmol, Eq: 1.2). The mixture was stirred for 1.5 hours and was then added at room temperature in one portion to a pre-stirred suspension of 2,8-diazaspiro[4.5]decane-1,3-dione hydrochloride (60 mg, 293 µmol, Eq: 1) and DIPEA (114 mg, 154 µl, 880 µmol, Eq: 3.0) in tetrahydrofuran (1 ml). The reaction mixture was stirred for 1.5 hours. LC/MS showed the reaction was finished. The reaction mixture was partitioned between ethyl acetate (30 ml) and 1 M aq. sodium carbonate (15 ml). The aqueous layer was extracted with one portion of ethyl acetate (30 ml). The combined organic layers were washed with one portion of brine (20 ml), dried over anhydrous sodium sulfate and concentrated *in vacuo.* The crude material was purified by preparative RP-HPLC followed by lyophilisation to afford 8-(benzo[d]thiazole-5-carbonyl)-2,8-diazaspiro[4.5]decane-1,3-dione (16 mg, 48.6 µmol, 16.6 %) as a white solid. MS (ISP): 330.1 ([M+H]⁺).

### Example 20

### (S)-8-(1,2,3,4-Tetrahydronaphthalene-1-carbonyl)-2,8-diazaspiro[4.5]decane-1,3-dione (OR ENANTIOMER)

### a) (R)-1,2,3,4-tetrahydronaphthalene-1-carbonyl chloride

To a solution of (R)-1,2,3,4-tetrahydronaphthalene-1-carboxylic acid (250 mg, 1.42 mmol, Eq: 1) in dichloromethane (11 ml) was added thionyl chloride (203 mg, 124 µl, 1.7 mmol, Eq: 1.2) at room temperature and a catalytic amount of *N*,*N*-dimethylformamide. The reaction mixture was stirred for 3 hours. The solvent was concentrated *in vacuo* to afford (R)-1,2,3,4-tetrahydronaphthalene-1-carbonyl chloride as a colorless oil (276 mg, 1.42 mmol, 99.9%), which was used in the next step without further purification.

### b) 8-(Benzo[d]thiazole-5-carbonyl)-2,8-diazaspiro[4.51decane-1,3-dione (MIXTURE OF 2 ENANTIOMERS)

To a suspension of 2,8-diazaspiro[4.5]decane-1,3-dione hydrochloride (60 mg, 293 µmol, Eq: 1) and triethylamine (89 mg, 123 µl, 880 µmol, Eq: 3.0) in tetrahydrofuran (1.5 ml) was added dropwise (*R*)-1,2,3,4-tetrahydronaphthalene-1-carbonyl chloride (62.8 mg, 322 µmol, Eq: 1.1) at 0 °C - 5 °C. The cooling bath was removed after 5 min and the reaction mixture was stirred for 15 hours. The reaction was quenched by addition of a solution of 2 M aq. sodium carbonate (5 ml). The reaction mixture was partitioned between ethyl acetate (30 ml) and a solution of 2 M aq. sodium carbonate (15 ml). The layers were separated. The aqueous layer was extracted with ethyl acetate (2 x 30 ml). The combined organic layers were washed with brine (20 ml), dried over anhydrous sodium sulfate, and concentrated *in vacuo.* Analytical chiral HPLC of the product revealed that some racemization had occurred during the reaction. The crude material was purified by flash column chromatography (silica gel, 4 g, 0% to 5% methanol in dichloromethane) to afford 8-(1,2,3,4-tetrahydronaphthalene-1-carbonyl)-2,8-diazaspiro[4.5]decane-1,3-dione (16 mg, 41.7 µmol, 14.2 %) as a white solid. MS (ISP): 327.1 ([M+H]⁺).

### c) (S)-8-(benzo[d]thiazole-5-carbonyl)-2,8-diazaspiro[4.51decane-1,3-dione (or ENANTIOMER)

The enantiomers of 8-(benzo[d]thiazole-5-carbonyl)-2,8-diazaspiro[4.5]decane-1,3-dione were separated using chiral HPLC (column: Reprosil Chiral-NR, eluent: heptane/ethanol + NH₄OAc (60:40), pressure: 18 bar; flow rate: 35 ml/min) affording (S)-8-(benzo[d]thiazole-5-carbonyl)-2,8-diazaspiro[4.5]decane-1,3-dione (or ENANTIOMER) (9.4 mg, white solid), retention time = 8.87 min. MS (ISP): 327.1 ([M+H]⁺).

### Example 21

### (R)-8-(1,2,3,4-Tetrahydronaphthalene-1-carbonyl)-2,8-diazaspiro[4.5]decane-1,3-dione (or ENANTIOMER)

The enantiomers of 8-(benzo[d]thiazole-5-carbonyl)-2,8-diazaspiro[4.5]decane-1,3-dione were separated using chiral HPLC (column: Reprosil Chiral-NR, eluent: heptane/ethanol + NH₄OAc (60:40), pressure: 18 bar; flow rate: 35 ml/min) affording (*R*)-8-(benzo[d]thiazole-5-carbonyl)-2,8-diazaspiro[4.5]decane-1,3-dione (or ENANTIOMER) (9.4 mg, white solid), retention time = 11.28 min. MS (ISP): 327.1 ([M+H]⁺).

### Example 22

### 8-(Pyrazolo[1,5-a]pyridine-3-carbonyl)-2,8-diazaspiro[4.5]decane-1,3-dione

To a suspension of pyrazolo[1,5-a]pyridine-3-carboxylic acid³¹ (40 mg, 247 µmol, Eq: 1) in tetrahydrofuran (0.5 ml) was added 1-chloro-*N*,*N*,2-trimethylpropenylamine (39.6 mg, 39.2 µl, 296 µmol, Eq: 1.2) at 0 °C - 5 °C. The reaction mixture was stirred at 0 °C for 30 min to afford a white suspension. The reaction mixture was stirred at room temperature for 15 min and was then added to a suspension of 2,8-diazaspiro[4.5]decane-1,3-dione hydrochloride (50.5 mg, 247 µmol, Eq: 1) and DIPEA (95.7 mg, 129 µl, 740 µmol, Eq: 3) in tetrahydrofuran (0.5 ml). The reaction mixture was stirred at room temperature for 30 min. The reaction mixture was poured into ethyl acetate/tetrahydrofuran (1:3) and extracted with brine. The organic layer was dried over Na₂SO₄ and concentrated *in vacuo.* The crude material was purified by flash chromatography (silica gel, 4 g, 0% to 10% MeOH in dichloromethane) to afford 8-(pyrazolo[1,5-a]pyridine-3-carbonyl)-2,8-diazaspiro[4.5]decane-1,3-dione (42 mg, 134 µmol, 54.5 %) as a white solid. MS (ISP): 313.1 ([M+H]⁺).

### Example 23

### 8-(Benzofuran-3-carbonyl)-2,8-diazaspiro[4.5]decane-1,3-dione

The title compound was obtained in analogy to example 22 using benzofuran-3-carboxylic acid³⁰ in place of pyrazolo[1,5-a]pyridine-3-carboxylic acid. Yellow solid. MS (ISP): 291.0 ([M+H]⁺).

### Example 24

### 8-(Benzo[d]thiazole-5-carbonyl)-1,3,8- triazaspiro[4.5]decane- 2,4-dione

The title compound was obtained in analogy to example 9 using benzo[d]thiazole-5-carboxylic acid³³ in place of benzofuran-3-carboxylic acid and stirring overnight instead of for 2 hours. White solid. MS (ISP): 331.1 ([M+H]⁺).
¹ Collins et al., Biochem J, 2017, 474(7), 1127-47
² WO2013020557
³ WO2013063560
⁴ WO 2013106643
⁵ WO2015160845
⁶ WO2016011906
⁷ WO2016105518
⁸ WO2017007612
⁹ WO2017024318
¹⁰ WO2017117473
¹¹ CAS 13625-39-3
¹² CAS 2079-25-6
¹³ CAS 13625-48-4
¹⁴ CAS 2696-03-9
¹⁵ CAS 26189-59-3
¹⁶ J. Chem. Soc., Chem. Commun. 1979, 1180; Org. Synth. 1980, 59, 26-34
¹⁷ CAS 615-20-3
¹⁸ CAS 152170-30-4
¹⁹ CAS 73781-91-6
²⁰ CAS 4548-45-2
²¹ CAS 350-46-9
²² Yen, H.-C. S., Xu, Q., Chou, D. M., Zhao, Z. & Elledge, S. J. Global Protein Stability Profiling in Mammalian Cells. Science 322, 918-923, doi: 10.1126/science. 1160489 (2008).
²³ Birdsall, B., King, R. W., Wheeler, M. R., Lewis, C. A. Jr, Goode, S. R., Dunlap, R. B. & Roberts, G. C. (1983). Anal. Biochem. 132, 353-361
²⁴ Eftink, Methods Enzymol. 1997;278:221-57
²⁵ CAS 85977-52-2
²⁶ CAS 23357-47-3
²⁷ CAS 65-85-0
²⁸ CAS 556-08-1
²⁹ CAS 587-48-4
³⁰ CAS 26537-68-8
³¹ CAS 16205-46-2
³² CAS 98-88-4
³³ CAS 68867-17-4

## Claims

1. A compound of formula I, or a pharmaceutically acceptable salt thereof, 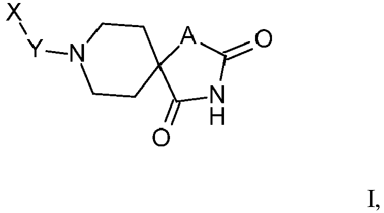 wherein
A is selected from the group consisting of
i.) NH, and
ii.) CH₂,
X is selected from the group consisting of
i.) aryl,
ii.) aryl substituted by R¹,
iii.) heteroaryl, and
iv.) heteroaryl substituted by R²;
Y is absent or -C(=O)-;
R¹ is selected from the group consisting of
i.) -COOH,
ii.) -NH-C(=O)-C₁₋₆-alkyl,
iii.) -NH₂, and
iv.) -NO₂;
R² is selected from the group consisting of
i.) -COOH,
ii.) -C(=O)-O-C₁₋₆-alkyl,
iii.) -NH₂, and
iv.) -NO₂.

2. A compound of formula I, or a pharmaceutically acceptable salt thereof, according to claim 1, wherein
X is selected from the group consisting of
i.) aryl, in particular 1,2,3,4-tetrahydronaphthalenyl or phenyl,
ii.) phenyl substituted by R¹,
iii.) heteroaryl, in particular benzo[d]thiazolyl, thiazolo[4,5-b]pyridinyl, benzofuranyl or pyrazolo[1,5-a]pyridinyl,
iv.) pyridinyl substituted by R²;
R¹ is selected from the group consisting of
i.) -NH-C(=O)-C₁₋₆-alkyl, in particular -NH-C(=O)-CH₃, and
ii.) -NO₂;
R² is selected from the group consisting of
i.) -C(=O)-O-C₁₋₆-alkyl, in particular -C(=O)-O-CH₃, and
ii.) -NO₂.

3. A compound of formula I, or a pharmaceutically acceptable salt thereof, according to anyone of claims 1-2, wherein
A is NH.

4. A compound of formula I, or a pharmaceutically acceptable salt thereof, according to anyone of claims 1-2, wherein
A is CH₂.

5. A compound of formula I, or a pharmaceutically acceptable salt thereof, according to anyone of claims 1-4, wherein
Y is absent.

6. A compound of formula I, or a pharmaceutically acceptable salt thereof, according to anyone of claims 1-4, wherein Y is -C(=O)-.

7. The compound of formula I, or pharmaceutically acceptable salts thereof, according to any one of claims 1-6, selected from the group consisting of
(*S*)-8-(1,2,3,4-tetrahydronaphthalene-1-carbonyl)-2,8-diazaspiro[4.5]decane-1,3-dione,
(*R*)-8-(1,2,3,4-tetrahydronaphthalene-1-carbonyl)-2,8-diazaspiro[4.5]decane-1,3-dione,
(*S*)-8-(1,2,3,4-tetrahydronaphthalene-1-carbonyl)-1,3,8-triazaspiro[4.5]decane-2,4-dione,
(*R*)-8-(1,2,3,4-tetrahydronaphthalene-1-carbonyl)-1,3,8-triazaspiro[4.5]decane-2,4-dione,
8-benzoyl-1,3,8-triazaspiro[4.5]decane-2,4-dione,
8-(benzo[*d*]thiazol-2-yl)-1,3,8-triazaspiro[4.5]decane-2,4-dione,
8-(thiazolo[4,5-*b*]pyridin-2-yl)-1,3,8-triazaspiro[4.5]decane-2,4-dione,
methyl 6-(2,4-dioxo-1,3,8-triazaspiro[4.5]decan-8-yl)nicotinate,
*N*-(4-(2,4-dioxo-1,3,8-triazaspiro[4.5]decane-8-carbonyl)phenyl)acetamide,
*N*-(3-(2,4-dioxo-1,3,8-triazaspiro[4.5]decane-8-carbonyl)phenyl)acetamide,
8-(benzofuran-3-carbonyl)-1,3,8-triazaspiro[4.5]decane-2,4-dione,
8-(pyrazolo[1,5-*a*]pyridine-3-carbonyl)-1,3,8-triazaspiro[4.5]decane-2,4-dione,
8-(5-nitropyridin-2-yl)-1,3,8-triazaspiro[4.5]decane-2,4-dione,
8-(4-nitrophenyl)-1,3,8-triazaspiro[4.5]decane-2,4-dione,
8-(benzo[*d*]thiazole-5-carbonyl)-1,3,8-triazaspiro[4.5]decane-2,4-dione,
8-(thiazolo[4,5-*b*]pyridin-2-yl)-2,8-diazaspiro[4.5]decane-1,3-dione,
8-(5-nitropyridin-2-yl)-2,8-diazaspiro[4.5]decane-1,3-dione,
8-(benzo[*d*]thiazol-2-yl)-2,8-diazaspiro[4.5]decane-1,3-dione,
8-(4-nitrophenyl)-2,8-diazaspiro[4.5]decane-1,3-dione,
8-benzoyl-2,8-diazaspiro[4.5]decane-1,3-dione,
8-(benzo[*d*]thiazole-5-carbonyl)-2,8-diazaspiro[4.5]decane-1,3-dione,
8-(benzofuran-3-carbonyl)-2,8-diazaspiro[4.5]decane-1,3-dione,
methyl 6-(1,3-dioxo-2,8-diazaspiro[4.5]decan-8-yl)nicotinate, and
8-(pyrazolo[1,5-a]pyridine-3-carbonyl)-2,8-diazaspiro[4.5]decane-1,3-dione.

8. A compound, or pharmaceutically acceptable salts thereof, having a chemical structure comprising:
P-L-C
wherein
L is a linker group;
C is a compound of formula I according to any one of the claims 1-7, wherein L is chemically linked to C; and
P is a protein targeting moiety that binds to a target protein or a target polypeptide, wherein L is chemically linked to P.

9. The compound according to claim 8, wherein L is selected from the group consisting of:
i) -NHCH₂-(CH₂)₁₋₃₀-C(=O)NH-, in particular -NHCH₂-(CH₂)₆₋C(=O)NH-, and
ii) -NH-(CH₂CH₂O)₁₋₂₅-C(=O)NH-.

10. The compound according to any one of claims 8-9, wherein P is a BRD4 inhibitor, in particular wherein P is

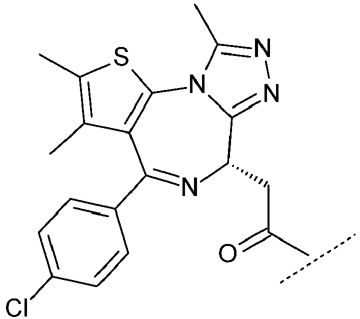

11. The compound according to any one of claims 1-10, or pharmaceutically acceptable salts thereof, for the therapeutic and/or prophylactic treatment of cancer.

12. Use of the compound according to any one of claims 1-10, or pharmaceutically acceptable salts thereof, for the therapeutic and/or prophylactic treatment of cancer.

13. Use of the compound according to any one of claims 1-10, or pharmaceutically acceptable salts thereof, for the preparation of a medicament for the therapeutic and/or prophylactic treatment of cancer.

14. A pharmaceutical composition comprising a compound according to any one of claims 1-10, and a therapeutically inert carrier.

15. A method for the therapeutic and/or prophylactic treatment of cancer, which method comprises administering an effective amount of a compound as defined in any one of claims 1-10.
